# EUROPÄISCHE PATENTSCHRIFT

(11) **EP 4 245 229 B1**
(45) Veröffentlichungstag und Bekanntmachung des Hinweises auf die Patenterteilung: **06.05.2026**
(21) Anmeldenummer: 23160108.9
(22) Anmeldetag: 06.03.2023
(51) Int. Cl.: A61B 17/14, A61B 17/15

(54) **CHIRURGISCHES INSTRUMENT**
SURGICAL INSTRUMENT
INSTRUMENT CHIRURGICAL

(30) Priorität: 08.03.2022 DE 102022202314
(43) Veröffentlichungstag der Anmeldung: 20.09.2023
(60) Teilanmeldung: 24164857.5
(73) Patentinhaber: Aesculap AG, 78532 Tuttlingen (DE)
(72) Erfinder: Firmbach, Franz-Peter, 78576 Emmingen-Liptingen (DE); Anhorn, Svenja, 72535 Heroldstatt (DE)
(74) Vertreter: Patentanwälte Ruff, Wilhelm, Beier, Dauster & Partner mbB

(56) Entgegenhaltungen:
- WO-A1-94/00056
- US-A- 5 925 049
- US-A1- 2014 364 857
- US-B2- 9 693 788

## Beschreibung

Die Erfindung befasst sich mit chirurgischen Instrumenten zur Verwendung bei einer Kniegelenkersatzoperation. Im Speziellen betrifft die Erfindung ein chirurgisches Instrument nach dem Oberbegriff des Anspruchs 1.

Die Verwendung von orthopädischen Prothesen als künstlicher Ersatz für beschädigte oder abgenutzte natürliche Knochenstrukturen ist gängige medizinische Praxis. Insbesondere Hüft- und Kniegelenkersatzoperationen gehören mittlerweile zum Standardrepertoire der chirurgischen Orthopädie.

Bei einer Kniegelenkersatzoperation (englisch: Total Knee Arthroplasty (TKA)) werden abgenutzte oder anderweitig durch Krankheit oder einen Unfall beeinträchtigte Gelenkflächen des Femurs und/oder der Tibia durch eine Kniegelenkprothese ersetzt. Solche Kniegelenkprothesen umfassen üblicherweise eine Femurkomponente, welche am distalen Ende des Femurs implantiert wird, und eine Tibiakomponente, welche am proximalen Ende der Tibia implantiert wird. Um eine einwandfreie Funktion des künstlichen Gelenkersatzes zu gewährleisten, müssen die besagten Komponenten hinsichtlich ihrer Lage und Orientierung in Bezug auf die Anatomie des Patienten und dessen Körperachsen in definierter Weise möglichst präzise positioniert werden. Andernfalls ist mit einem für den Patienten nicht zufriedenstellenden Ergebnis zu rechnen. Hinsichtlich der Positionierung der Komponenten existieren unterschiedliche chirurgische Ansätze.

Ein als Mechanical Alignment bekannter und bislang vorwiegend verwendeter Ansatz sieht vor, dass die Position und Ausrichtung der künstlichen Gelenkachsen der Kniegelenkprothese mechanisch ideal und insoweit ohne Berücksichtigung etwaiger orthopädischer Fehlstellungen des Patienten festgelegt werden. Hierbei dient oftmals die Längsachse der Tibia als Referenzachse für die Ausrichtung und Positionierung. Klinische Studien haben gezeigt, dass der Mechanical Alignment-Ansatz zu einer als unnatürlich empfundenen Funktion des künstlichen Kniegelenks führen kann.

Als ein weiterer Ansatz ist das sog. Kinematic Alignment bekannt. Bei dieser Vorgehensweise werden die Femurkomponente und die Tibiakomponente unter Berücksichtigung etwaiger orthopädischer Fehlstellungen des Patienten positioniert. Ziel hierbei ist es, die natürliche und unter Umständen mit Fehlstellungen behaftete Gelenkausrichtung des Patienten wiederherzustellen. Klinische Studien haben gezeigt, dass der Kinematic Alignment-Ansatz oftmals mit einer verbesserten Patientenzufriedenheit einhergeht. Insbesondere die Funktion des künstlichen Kniegelenks wird seitens der Patienten als eher natürlich empfunden. Zusätzlich zu der präzisen Positionierung ist es wichtig, dass die Femur- und die Tibiakomponente jeweils in einer für die Anatomie des Patienten passenden Größe ausgewählt und implantiert werden. Die Größe der Femurkomponente richtet sich hierbei im weitesten Sinne nach der Größe des Femurs, die intraoperativ mit einem hierfür bestimmten Messinstrument ermittelt wird. Solche Messinstrumente werden auch als femoral sizer oder femoral sizing system bezeichnet.

Es ist üblich, dass die Größenermittlung nach der Resektion des distalen Femurs erfolgt. Hierbei wird das bekannte Messinstrument an die resezierte Stirnfläche des distalen Femurs angelegt und in Bezug auf anatomische Referenzflächen oder -punkte (englisch: anatomical landmarks) ausgerichtet. Nach erfolgter Größenermittlung wird der distale Femur weitergehend zur Anbringung der Femurkomponente vorbereitet. Dies erfordert eine weitergehende Resektion, die üblicherweise einen anterioren Schnitt, einen posterioren Schnitt und sog. Fasenschnitte (englisch: chamfer cuts) umfasst. Die besagten Schnitte werden unter Verwendung eines an der distalen Stirnfläche des Femurs befestigten Schnittblocks (englisch: cutting jig) ausgeführt. Für unterschiedliche Femurgrößen sind üblicherweise unterschiedliche Schnittblöcke mit unterschiedlichen anterior-posterioren und/oder mediolateralen Abmessungen vorhanden. Die jeweils erforderliche Abmessung wird in Abhängigkeit der zuvor ermittelten Größe des Femurs seitens des Operateurs ausgewählt. Die separate Ermittlung der Größe des Femurs, die hierauf basierende Auswahl des passenden Schnittblocks und dessen femurseitige Anbringung können mit einem erhöhten Zeit- und instrumentellen Aufwand einhergehen und bergen potenzielle Fehlerquellen.

Mit dem Bestreben, die Patientenzufriedenheit weiter zu verbessern, geht ein grundsätzlicher Bedarf nach möglichst präzisen, einfach verwendbaren und kosteneffizienten chirurgischen Instrumenten zur Umsetzung des Kinematic Alignment einher. Die vorliegende Erfindung befasst sich mit einem solchen chirurgischen Instrument und entsprechenden Instrumentensystemen.

Die WO 94/00056 A1 offenbart ein chirurgisches Instrument nach dem Oberbegriff des Anspruchs 1.

Zudem ist aus der US 2014/0364857 A1 ein chirurgisches Instrument bekannt.

Aufgabe der Erfindung ist es, ein chirurgisches Instrument zur Verwendung bei einer Kniegelenkersatzoperation bereitzustellen, das die mit dem Stand der Technik einhergehenden Nachteile beseitigt oder vermindert und insbesondere Zeit- und/oder instrumentelle Einsparungen ermöglicht.

Diese Aufgabe wird durch das Bereitstellen eines chirurgischen Instruments mit den Merkmalen des Anspruchs 1 gelöst.

Das erfindungsgemäße chirurgische Instrument weist auf: einen Schnittblock mit einer proximal orientierten Blockrückseite, welche zur Anlage an einer distalen Stirnfläche eines resezierten Femurs eingerichtet ist, und mit wenigstens einem von der Blockrückseite auf eine distal gegenüberliegende Blockvorderseite reichenden sowie mediolateral längserstreckten Führungsschlitz, welcher zur Aufnahme und Führung eines Sägeblatts eingerichtet ist, einen Referenzfuß, welcher im Bereich einer posterioren Blockunterseite des Schnittblocks an demselben anbringbar oder angebracht ist und eine anterior orientierte Fußoberseite aufweist, welche - in einem angebrachten Zustand des Referenzfußes - proximal über die Blockrückseite ragt und zur Anlage an posterioren Kondylen des Femurs eingerichtet ist, und einen Taster, welcher im Bereich einer der Blockunterseite anterior gegenüberliegenden Blockoberseite des Schnittblocks an demselben anbringbar oder angebracht ist und eine Tasterspitze aufweist, welche - in einem angebrachten Zustand des Tasters - proximal über die Blockrückseite ragt und zur Anlage an einer anterioren Fläche des Femurs eingerichtet ist. Durch die erfindungsgemäße Lösung kann auf eine gesonderte Größenmessung und/oder -kontrolle und ein hierfür üblicherweise notwendiges gesondertes Messinstrument verzichtet werden. Hierdurch können Zeit, instrumenteller Aufwand und damit einhergehend Kosten eingespart werden. Das erfindungsgemäße chirurgische Instrument ermöglicht zum einen die erforderliche Größenmessung und/oder -kontrolle des Femurs. Zu diesem Zweck sind insbesondere der Referenzfuß und der Taster vorhanden und an dem Schnittblock angebracht oder anbringbar. Das erfindungsgemäße chirurgische Instrument ermöglicht zum anderen die zur weiteren Resektion des distalen Femurs notwendige Schnittführung. Zu diesem Zweck ist der Schnittblock vorhanden und weist den besagten wenigstens einen Führungsschlitz zur Aufnahme und Führung des Sägeblatts auf. Der Schnittblock kann auch als Sägeblock oder cutting jig bezeichnet werden. Bei einer bevorzugten Ausgestaltung weist der Schnittblock mehrere Führungsschlitze auf, vorzugsweise einen anterioren Führungsschlitz, einen posterioren Führungsschlitz sowie zwei Fasenführungsschlitze, so dass auch von einem 4-in-1-Schnittblock gesprochen werden kann. Die Blockrückseite dient einer distalen Referenzierung. In der Verwendung des chirurgischen Instruments kontaktiert die Blockrückseite die distale Stirnfläche des resezierten Femurs. Die Fußoberseite dient einer posterioren Referenzierung. In der Verwendung des chirurgischen Instruments kontaktiert die Fußoberseite die posterioren Kondylen des Femurs. Der Taster, genauer: dessen Tasterspitze, dient einer anterioren Referenzierung. In der Verwendung des chirurgischen Instruments kontaktiert die Tasterspitze die besagte anteriore Fläche des Femurs. Der Taster kann auch als Stylus bezeichnet werden. Bei einer Ausgestaltung ist die Tasterspitze relativ zu dem Schnittblock, vorzugsweise proximodistal linearbeweglich und/oder um eine anteroposterior ausgerichtete Schwenkachse schwenkbeweglich, verlagerbar. Bei einer weiteren Ausgestaltung ist die Tasterspitze relativ zu dem Schnittblock unbeweglich.

Die in dieser Beschreibung verwendeten Lage- und Richtungsbezeichnungen beziehen sich auf den Körper eines Patienten, insbesondere dessen Femur, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximaldistale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnung wegen werden nachfolgend in erster Linie die besagten anatomischen Lage- und Richtungsbezeichnungen verwendet. Weiter werden Bezeichnungen wie "Rückseite" einer Komponente oder eines Abschnitts des chirurgischen Instruments, beispielsweise des Schnittblocks, in Bezug auf eine proximal gerichtete Blickrichtung verwendet. Dementgegen werden Bezeichnungen wie "Vorderseite" in Bezug auf eine distal gerichtete Blickrichtung verwendet.

In Ausgestaltung der Erfindung weist der Schnittblock eine posterior angeordnete erste Befestigungsaufnahme auf, der Referenzfuß weist ein komplementäres Befestigungselement auf, und die erste Befestigungsaufnahme und das Befestigungselement sind lösbar form- und/oder kraftschlüssig miteinander verbindbar oder verbunden. Die erste Befestigungsaufnahme und das Befestigungselement bilden bei unterschiedlichen Ausgestaltungen unterschiedliche Arten von Fügeverbindungen aus. Bei einer Ausgestaltung liegt eine lösbare Steckverbindung vor. Bei anderen Ausgestaltungen liegt eine Rast-, Klemm- und/oder Schnappverbindung oder dergleichen vor. Denkbar ist zudem eine Schraubverbindung. Die lösbare Verbindung zwischen dem Schnittblock und dem Referenzfuß ermöglicht, dass letzterer nach erfolgter Größenkontrolle von dem Schnittblock gelöst und entfernt werden kann. Hernach können entsprechende Schnitte unter Verwendung des Schnittblocks ausgeführt werden.

In weiterer Ausgestaltung der Erfindung ist die erste Befestigungsaufnahme ein von der Blockrückseite auf die Blockvorderseite reichender sowie mediolateral gerade längserstreckter Aufnahmeschlitz, und das Befestigungselement ist ein ebenes plattenförmiges Steckelement, welches zum form- und/oder kraftschlüssigen Einstecken in den Aufnahmeschlitz eingerichtet ist. Zur Befestigung des Referenzfußes wird das Steckelement ausgehend von der Blockvorderseite in proximaler Richtung in den Aufnahmeschlitz eingesteckt. Das Steckelement weist eine zu dem Aufnahmeschlitz komplementäre Geometrie auf und umgekehrt. In eingestecktem Zustand ist das Steckelement form- und/oder kraftschlüssig in dem Aufnahmeschlitz gehalten. Hierdurch wird eine einfache und dennoch robuste Befestigung des Referenzfußes erreicht.

In weiterer Ausgestaltung der Erfindung weist das Steckelement wenigstens einen anteroposterior erstreckten Trennspalt auf, der unter Ausbildung eines elastisch federbeweglichen Federzungenabschnitts im Wesentlichen U-förmig längserstreckt ist, wobei der Federzungenabschnitt - in dem angebrachten Zustand des Referenzfußes - federkraftbelastet gegen eine Innenwand des Aufnahmeschlitzes drückt. Hierdurch wird eine erhöhte Reibung zwischen dem Referenzfuß, genauer: dem Federzungenabschnitt, und dem Aufnahmeschlitz, genauer: dessen Innenwand, erreicht. Dies führt zu einer verbesserten Befestigung des Referenzfußes. Der Federzungenabschnitt ist aufgrund der im Wesentlichen U-förmigen Längserstreckung des Trennspalts zungenförmig und/oder weist eine Zungenform auf. Der Trennspalt ist anteroposterior erstreckt und reicht folglich ausgehend von einer anterior orientierten Oberseite des Steckelements durchgängig bis auf eine posterior orientierte Unterseite des Steckelements. Der Federzungenabschnitt drückt bei einer Ausgestaltung ausgehend von der Oberseite des Steckelements (anterior) und bei einer weiteren Ausgestaltung ausgehend von der Unterseite des Steckelements (posterior) gegen die Innenwand des Aufnahmeschlitzes.

In weiterer Ausgestaltung der Erfindung weist der Aufnahmeschlitz zwei getrennte Schlitzabschnitte auf, welche in mediolateraler Richtung durch einen Steg voneinander getrennt sind, und das Steckelement weist zwei getrennte Steckabschnitte auf, welche in mediolateraler Richtung durch einen Spalt voneinander getrennt sind. In befestigtem Zustand des Referenzfußes taucht der Steg in den Spalt ein und führt zu einer weiter verbesserten Befestigung. Dies insbesondere in mediolateraler Richtung. Bei dieser Ausgestaltung der Erfindung kann auch von einem ersten Schlitzabschnitt und einem zweiten Schlitzabschnitt und dementsprechend von einem ersten Steckabschnitt und einem zweiten Steckabschnitt gesprochen werden.

In weiterer Ausgestaltung der Erfindung ist der Aufnahmeschlitz - in einem nicht angebrachten Zustand des Referenzfußes - zur Aufnahme und Führung des Sägeblatts eingerichtet und bildet einen posterioren Führungsschlitz des Schnittblocks. Dies ist eine besonders bevorzugte Ausgestaltung der Erfindung. Bei dieser Ausgestaltung kommt dem Aufnahmeschlitz eine besonders vorteilhafte Mehrfachfunktion zu. Zum einen dient der Aufnahmeschlitz der Aufnahme des Steckelements zwecks lösbarer Befestigung des Referenzfußes an dem Schnittblock. Zum anderen dient der Aufnahmeschlitz gleichsam als Führungsschlitz für das Sägeblatt. Hierdurch kann ein besonders einfacher und kompakter Aufbau des Schnittblocks erreicht werden.

In weiterer Ausgestaltung der Erfindung ist die erste Befestigungsaufnahme eine in die Blockvorderseite proximal eingesenkte Aufnahmetasche und das Befestigungselement ist zum Ausbilden einer Rast- und/oder Klemmverbindung mit der Aufnahmetasche eingerichtet. Dementsprechend ist das Befestigungselement bei einer Ausgestaltung ein Rastelement, bei einer weiteren Ausgestaltung ein Klemmelement.

In weiterer Ausgestaltung der Erfindung weist der Referenzfuß einen posterior von dem Befestigungselement beabstandeten sowie ebenen plattenförmigen Fortsatz auf, dessen Oberseite die Fußoberseite bildet. In befestigtem Zustand des Referenzfußes ist der Fortsatz unterhalb, d.h. posterior beabstandet, an der Blockunterseite angeordnet. Zu diesem Zweck ist der Fortsatz posterior von dem Befestigungselement beabstandet. Die Oberseite des Fortsatzes ist anterior orientiert und zur Anlage an den posterioren Kondylen des Femurs eingerichtet. Der Fortsatz ragt in proximaler Richtung über die Blockrückseite. Bei einer Ausgestaltung weist der Fortsatz zwei getrennte Fortsatzabschnitte auf, welche in mediolateraler Richtung durch einen Spalt voneinander getrennt sind. Bei dieser Ausgestaltung kann auch von einem ersten Fortsatzabschnitt und einem zweiten Fortsatzabschnitt oder alternativ von einem medialen Fortsatzabschnitt und einem lateralen Fortsatzabschnitt gesprochen werden. Der mediale Fortsatzabschnitt ist zur Referenzierung an der medialen posterioren Kondyle vorgesehen. Der laterale Fortsatzabschnitt ist dementsprechend zur Referenzierung an der lateralen posterioren Kondyle vorgesehen.

In weiterer Ausgestaltung der Erfindung weist der Referenzfuß einen distal angeordneten Griffabschnitt auf, von welchem das Befestigungselement und/oder der Fortsatz in proximaler Richtung abragt, wobei der Griffabschnitt eine lateral orientierte erste Grifffläche, eine medial orientierte zweite Grifffläche und eine proximal orientierte Anschlagfläche aufweist. Der Griffabschnitt erlaubt eine ergonomische manuelle Handhabung des Referenzfußes durch den Operateur. Zu diesem Zweck ist der Griffabschnitt distal an dem Referenzfuß angeordnet. In befestigtem Zustand des Referenzfußes ist der Griffabschnitt insoweit leicht zugänglich auf der Blockvorderseite angeordnet. Der Griffabschnitt kann zwischen den Fingern einer Hand gegriffen werden und weist zu diesem Zweck die beiden Griffflächen auf. Die lateral orientierte erste Grifffläche ist lateral angeordnet und kann somit auch als laterale Grifffläche bezeichnet werden. Die medial orientierte zweite Grifffläche ist medial angeordnet und kann folglich auch als mediale Grifffläche bezeichnet werden. Die proximal orientierte Anschlagfläche ist besonders vorteilhaft in Verbindung mit Ausführungsformen, bei welchen das Befestigungselement des Referenzfußes als plattenförmiges Steckelement ausgebildet ist. In diesem Fall begrenzt die proximal orientierte Anschlagfläche die Einstecktiefe des Steckelements in den Aufnahmeschlitz.

Weiter gemäß der Erfindung weist der Schnittblock eine anterior angeordnete zweite Befestigungsaufnahme auf, der Taster weist ein komplementäres Befestigungselement auf und die zweite Befestigungsaufnahme und das Befestigungselement des Tasters sind lösbar form- und/oder kraftschlüssig miteinander verbindbar oder verbunden. Hierdurch kann der Taster nach erfolgter Größenmessung und/oder -kontrolle auf einfache Weise von dem Schnittblock gelöst und entfernt werden. Hiernach kann die weitere Resektion des distalen Femurs unter Verwendung des Schnittblocks erfolgen.

In weiterer Ausgestaltung der Erfindung ist die zweite Befestigungsaufnahme als Bestandteil eines Universalbefestigungssystems gestaltet und zur Verbindung mit weiteren Instrumentenkomponenten, bevorzugt einer chirurgischen Navigationsvorrichtung, eingerichtet. Die weitere Instrumentenkomponente weist folglich ebenfalls ein komplementäres Befestigungselement auf. Das Universalbefestigungssystem ermöglicht ein wahlweises Befestigen und Lösen unterschiedlicher Instrumentenkomponenten an dem Schnittblock. Insbesondere können der Taster und alternativ wenigstens eine weitere Instrumentenkomponente, insbesondere die besagte chirurgische Navigationsvorrichtung, an dem Schnittblock lösbar befestigt werden.

Weiter gemäß der Erfindung weist die zweite Befestigungsaufnahme eine Rastaufnahme auf, welche in die Blockoberseite posterior eingesenkt, mediolateral längserstreckt sowie medial oder lateral einends offen ist, und das Befestigungselement des Tasters weist einen komplementären Rastabschnitt auf, welcher unter Ausbildung einer lösbaren Schnapp-Verbindung mit der Rastaufnahme des Schnittblocks zusammenwirkt. Die Rastaufnahme ist mediolateral längserstreckt und bei einer Ausgestaltung in Richtung einer medialen Blockaußenseite offen und bei einer weiteren Ausgestaltung in Richtung einer lateralen Blockaußenseite offen. Je nachdem kann der komplementäre Rastabschnitt in lateraler oder in medialer Richtung in die Rastaufnahme eingeschoben und unter Ausbildung der Schnapp-Verbindung mit derselben verbunden werden. Die Rastaufnahme ist zudem in die Blockoberseite eingesenkt und insoweit in anteriorer Richtung offen. Hierdurch ist die Schnapp-Verbindung für den ausführenden Operateur besonders einfach und zuverlässig einsehbar. Somit kann leicht erkannt werden, ob die Schnapp-Verbindung anforderungsgemäß ausgebildet ist oder nicht.

In weiterer Ausgestaltung der Erfindung weist die zweite Befestigungsaufnahme eine Steckaufnahme auf, welche mediolateral in eine Blockaußenseite des Schnittblocks längserstreckt ist, und das Befestigungselement des Tasters weist einen komplementären Steckabschnitt auf, welcher unter Ausbildung einer lösbaren Steckverbindung mit der Steckaufnahme zusammenwirkt. Vorzugsweise weist die Steckaufnahme eine zylindrische, bevorzugt kreiszylindrische, Querschnittsform auf. Entsprechendes gilt sinngemäß im Hinblick auf die Querschnittsform des komplementären Steckabschnitts des Tasters. Der Steckabschnitt kann auch als Stift, Bolzen und/oder Zapfen bezeichnet werden. Dementsprechend kann die Steckaufnahme auch als Bohrung, Stift-, Zapfen- und/oder Bolzenaufnahme bezeichnet werden. Bei einer Ausgestaltung ist die Steckaufnahme ausgehend von der lateralen Blockaußenseite des Schnittblocks in medialer Richtung in dieselbe hinein längserstreckt. Bei einer weiteren Ausgestaltung ist die Steckaufnahme dementgegen ausgehend von einer medialen Blockaußenseite in lateraler Richtung in dieselbe hinein längserstreckt. Diese Ausgestaltung der Erfindung ist besonders vorteilhaft in Kombination mit Ausgestaltungen, bei welchen die zweite Befestigungsaufnahme zusätzlich eine Rastaufnahme aufweist. In diesem Fall dient die Steckaufnahme einer verbesserten Befestigung. Dabei ist die Steckaufnahme vorzugsweise posterior von der Rastaufnahme beabstandet an dem Schnittblock angeordnet.

In weiterer Ausgestaltung der Erfindung sind mehrere unterschiedliche Kompensationselemente mit unterschiedlicher anteroposteriorer Dicke vorhanden, wobei die unterschiedlichen Kompensationselemente jeweils an der Fußoberseite anbringbar, vorzugsweise auf dieselbe aufsteckbar und zur Kompensation unterschiedlich stark ausgeprägter Abnutzungen der posterioren Kondylen eingerichtet sind. Die unterschiedlichen Kompensationselemente dienen vereinfacht ausgedrückt einer Korrektur der Orientierung des Schnittblocks in Bezug auf die Längsachse des Femurs. Mit anderen Worten ausgedrückt, kann mittels der unterschiedlichen Kompensationselemente eine Rotation des Schnittblocks um eine proximodistale Achse beeinflusst werden. Der Kinematic Alignment-Ansatz sieht üblicherweise eine sog. 0°-Orientierung vor. Um eine 0°-Orientierung zu gewährleisten, ist eine Kompensation etwaiger Abnutzungen der posterioren Kondylen erforderlich. Die Abnutzungen können naturgemäß unterschiedlich stark ausgeprägt sein. Dementsprechend sind mehrere unterschiedlich dimensionierte, genauer: dicke, Kompensationselemente vorhanden. Je stärker der Grad der Abnutzung, desto dicker das erforderliche Kompensationselement. Die unterschiedlichen Kompensationselemente sind jeweils an der Fußoberseite anbringbar und zur unmittelbaren Kontaktierung der posterioren Kondylen eingerichtet. Vorzugsweise umfassen die mehreren unterschiedlichen Kompensationselemente laterale Kompensationselemente zur Kontaktierung der lateralen posterioren Kondyle und mediale Kompensationselemente zur Kontaktierung der medialen posterioren Kondyle.

Die Erfindung betrifft zudem ein chirurgisches Instrumentensystem zur Verwendung bei einer Kniegelenkersatzoperation mit wenigstens einem chirurgischen Instrument nach der vorhergehenden Beschreibung, wobei der Referenzfuß und der Taster jeweils lösbar an dem Schnittblock angebracht sind, und mit weiteren unterschiedlichen Schnittblöcken, welche sich im Hinblick auf wenigstens eine, insbesondere anteroposteriore und/oder mediolaterale, Abmessung unterscheiden, wobei der Referenzfuß und der Taster jeweils an jedem der weiteren unterschiedlichen Schnittblöcke anbringbar sind. In der Verwendung des chirurgischen Instrumentensystems erfolgt die Auswahl des jeweils passenden Schnittblocks beispielsweise auf der Grundlage einer präoperativen Untersuchung, Röntgenbildern oder dergleichen. Nach der Vorauswahl des Schnittblocks aus den mehreren unterschiedlichen Schnittblöcken können der Referenzfuß und der Taster an dem Schnittblock angebracht werden. Dabei dienen der Referenzfuß und der Taster in erster Linie einer Kontrolle der zuvor erfolgten Vorauswahl der Größe des Schnittblocks. Zu diesem Zweck wird die Fußoberseite auf die vorbeschriebene Weise an den posterioren Kondylen des Femurs angelegt. Die Blockrückseite wird an der distalen Stirnfläche des resezierten Femurs angelegt. Die eigentliche Kontrolle erfolgt sodann mittels der Tasterspitze. Sofern diese unter entsprechender Anlage der Fußoberseite und der Blockrückseite gleichzeitig eine anteriore Fläche des Femurs, in dem Bereich, an welchem das sog. anteriore Schild der zu implantierenden Femurkomponente später zum Liegen kommt, kontaktiert, kann von einer korrekten Vorauswahl der Größe des Schnittblocks ausgegangen werden.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivdarstellung eine Ausführungsform eines erfindungsgemäßen chirurgischen Instruments mit einem Schnittblock, einem Referenzfuß und einem Taster,
- Fig. 2: eine weitere schematische Perspektivdarstellung des chirurgischen Instruments nach Fig. 1,
- Fig. 3, 4: das chirurgische Instrument nach den Fig. 1 und 2 in einer schematischen Aufsicht (Fig. 3) und einer schematischen Seitenansicht (Fig. 4),
- Fig. 5: in schematischer Perspektivdarstellung eine exemplarische intraoperative Situation, in welcher das chirurgische Instrument nach den Fig. 1 bis 4 an einem distalen Femur angeordnet ist,
- Fig. 6: die intraoperative Situation nach Fig. 5 in einer weiteren schematischen Perspektivdarstellung,
- Fig. 7, 8: der Schnittblock des chirurgischen Instruments nach den Fig. 1 bis 6 in unterschiedlichen schematischen Perspektivdarstellungen mit Blickrichtung auf eine Blockvorderseite (Fig. 7) und eine Blockrückseite (Fig. 8),
- Fig. 9: in schematischer Perspektivdarstellung der Referenzfuß des chirurgischen Instruments nach den Fig. 1 bis 6,
- Fig. 10: eine schematische Seitenansicht des Referenzfußes nach Fig. 9,
- Fig. 11: in schematischer Perspektivdarstellung der Taster des chirurgischen Instruments nach den Fig. 1 bis 6,
- Fig. 12: eine schematische Längsschnittdarstellung des Tasters nach Fig. 11 entlang eines Schnitts B-B gemäß Fig. 13,
- Fig. 13: eine schematische Aufsicht des Tasters nach den Fig. 11 und 12,
- Fig. 14: in schematischer Perspektivdarstellung eine weitere Ausführungsform eines erfindungsgemäßen chirurgischen Instruments,
- Fig. 15: in schematischer Perspektivdarstellung mit Blickrichtung auf eine Blockvorderseite den Schnittblock des chirurgischen Instruments nach Fig. 14,
- Fig. 16: in schematischer Perspektivdarstellung den Referenzfuß des chirurgischen Instruments nach Fig. 14,
- Fig. 17: in schematisch stark vereinfachter Prinzipdarstellung eine Ausführungsform eines erfindungsgemäßen chirurgischen Instrumentensystems und
- Fig. 18: in einer der Fig. 17 entsprechenden Darstellungsweise ein nicht erfindungsgemäßes chirurgisches Instrumentensystem.

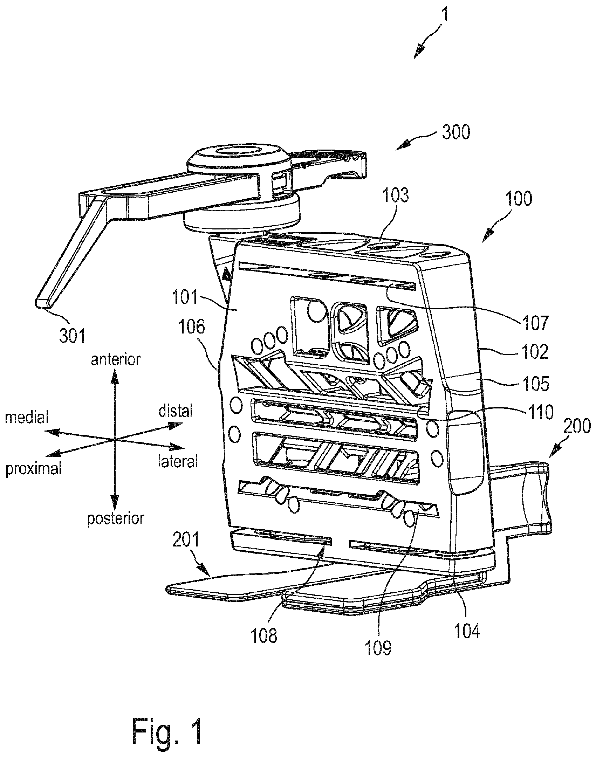

Gemäß den Fig. 1 bis 6 ist ein chirurgisches Instrument 1 zur Verwendung bei einer Kniegelenkersatzoperation vorgesehen und weist einen Schnittblock 100, einen Referenzfuß 200 und einen Taster 300 auf.

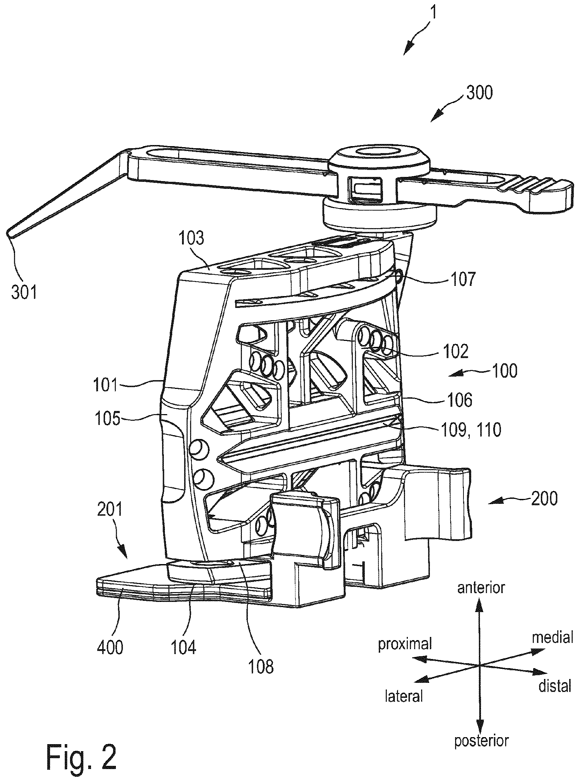

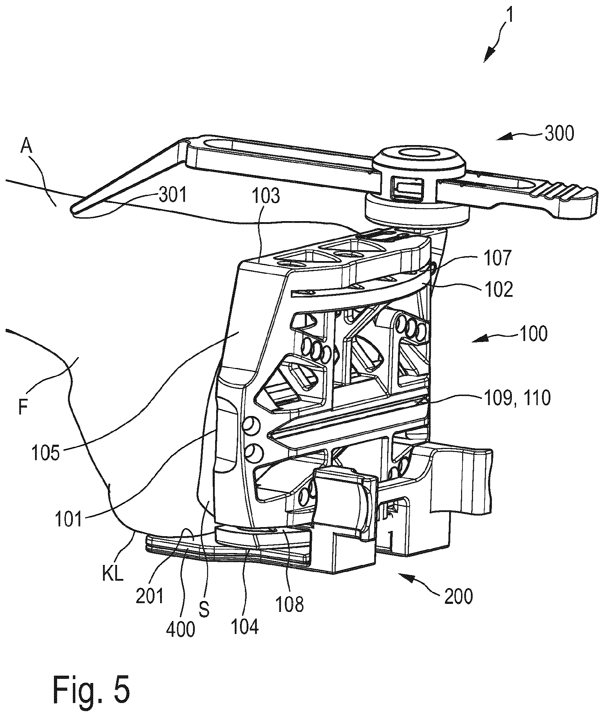

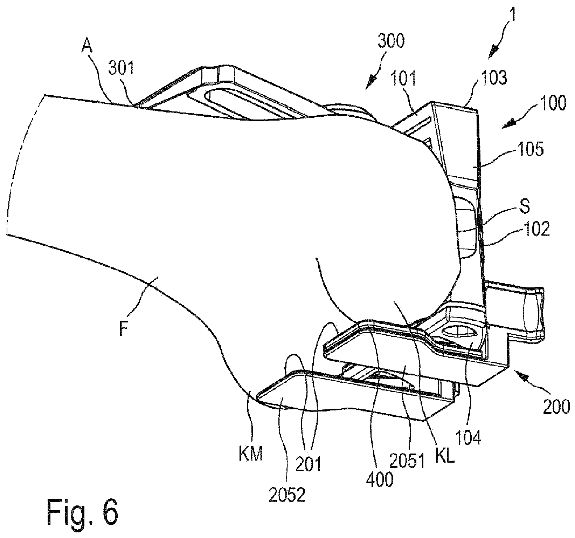

Der Schnittblock 100 weist eine proximal orientierte Blockrückseite 101, eine distal gegenüberliegende Blockvorderseite 102, eine anteriore Blockoberseite 103, eine posteriore Blockunterseite 104, eine laterale Blockaußenseite 105 und eine mediale Blockaußenseite 106 auf. Die Blockrückseite 101 ist zur Anlage an einer distalen Stirnfläche S eines resezierten Femurs F eingerichtet (siehe Fig. 5, 6). Eine Normalenrichtung der Blockrückseite 101 ist parallel zu der in den Fig. 1 und 2 eingezeichneten proximodistalen Achse orientiert und weist in proximale Richtung. Der Schnittblock 100 weist zudem wenigstens einen Führungsschlitz 107 auf. Vorliegend sind mehrere Führungsschlitze vorgesehen, nämlich der besagte Führungsschlitz 107, der auch als anteriorer Führungsschlitz 107 bezeichnet werden kann, ein posteriorer Führungsschlitz 108, sowie Fasenführungsschlitze 109, 110. Die Führungsschlitze 107, 108, 109, 110 reichen jeweils durchgängig von der Blockvorderseite 102 auf die Blockrückseite 101. Zudem sind die Führungsschlitze 107, 108, 109, 110 jeweils mediolateral, d.h. parallel zu der in den Fig. 1 und 2 eingezeichneten mediolateralen Achse längserstreckt. Die Führungsschlitze 107, 108, 109, 110 sind jeweils zur Aufnahme und Führung eines zeichnerisch nicht näher dargestellten Sägeblatts eingerichtet.

Der Referenzfuß 200 ist im Bereich der posterioren Blockunterseite 104 an dem Schnittblock angebracht und weist eine anterior orientierte Fußoberseite 201 auf. Eine nicht näher eingezeichnete Flächennormale der Fußoberseite 201 ist folglich parallel zu der in den Fig. 1 und 2 eingezeichneten anteroposterioren Achse ausgerichtet und weist in anteriore Richtung. Die Fußoberseite 201 ragt in proximaler Richtung über die Blockrückseite 101 hinaus und ist zur Anlage an posterioren Kondylen KL, KM des Femurs F eingerichtet (siehe Fig. 5, 6). Bei der vorliegenden Ausführungsform ist der Referenzfuß 200 auf noch näher beschriebene Weise lösbar mit dem Schnittblock 100 verbunden.

Der Taster 300 ist im Bereich der Blockoberseite 103 an dem Schnittblock 100 angebracht und weist eine Tasterspitze 301 auf. Die Tasterspitze 301 ragt in proximaler Richtung über die Blockrückseite 101 hinaus und ist zur Anlage an einer anterioren Fläche A des Femurs F eingerichtet (siehe Fig. 5, 6). Bei der vorliegenden Ausführungsform ist der Taster 300 auf noch näher beschriebene Weise lösbar mit dem Schnittblock 100 verbunden.

Das chirurgische Instrument 1 dient mehreren Zwecken. Zum einen kann mittels des chirurgischen Instruments 1 eine Größenmessung und/oder -kontrolle an dem distal resezierten Femur F durchgeführt werden. Zum anderen dient das chirurgische Instrument einer Führung des besagten Sägeblatts bei der Resektion des Femurs.

Zur Größenmessung und/oder -kontrolle wird die Blockrückseite 101 in Kontakt mit der Stirnfläche S gebracht. Gleichzeitig wird die Fußoberseite 201 in Kontakt mit den posterioren Kondylen KL, KM gebracht. Hierdurch wird das chirurgische Instrument in Bezug auf die anteroposteriore Achse und die proximodistale Achse des Femurs F ausgerichtet. Die eigentliche Größenmessung und/oder -kontrolle erfolgt mittels des Tasters 300 und in Abhängigkeit davon, ob dessen Tasterspitze 301 bei gegebener Ausrichtung des chirurgischen Instruments 1 an der Stirnfläche S und den posterioren Kondylen KL, KM die anteriore Fläche A des Femurs F kontaktiert.

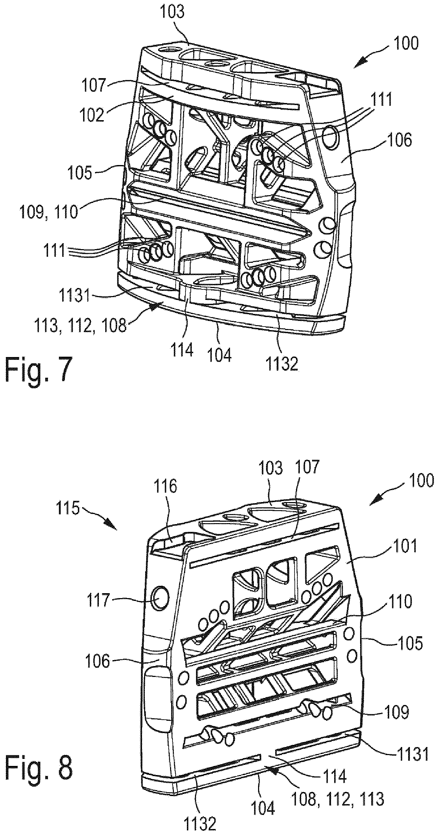

Nach erfolgter Größenmessung und/oder -kontrolle wird der Schnittblock 100 auf eine dem Fachmann bekannte Weise femurseitig fixiert, beispielsweise mittels hierfür geeigneter Pins, Schrauben oder dergleichen. Diese können in hierfür vorgesehene Befestigungsöffnungen 111 des Schnittblocks (siehe Fig. 7, 8) eingebracht werden. Nach der femurseitigen Befestigung des Schnittblocks 100 erfolgt die weitergehende Resektion des Femurs F. Hierfür wird das Sägeblatt auf eine dem Fachmann bekannte Weise durch die Führungsschlitze 107, 108, 109, 110 geführt und der Femur entsprechend reseziert. Bei der vorliegend gezeigten Ausführungsform werden zuvor sowohl der Referenzfuß 200 als auch der Taster 300 von dem Schnittblock 100 gelöst und entfernt.

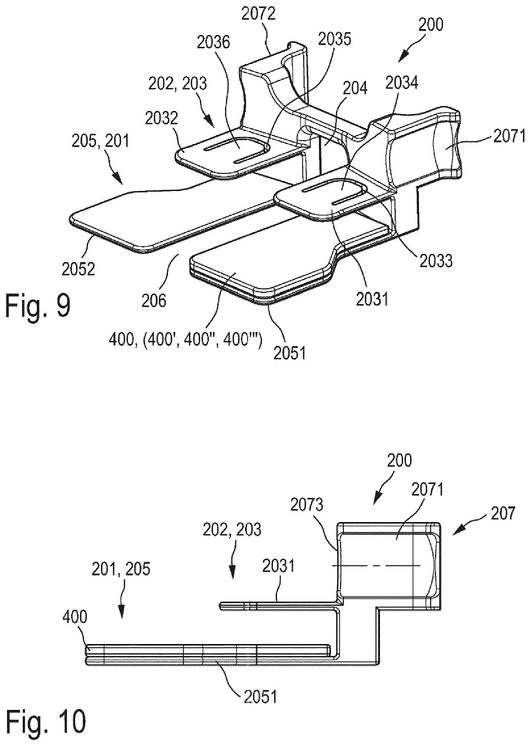

Bei der gezeigten Ausführungsform sind der Schnittblock 100 und der Referenzfuß 200 mittels einer lösbaren kraft- und/oder formschlüssigen Fügeverbindung miteinander verbunden. Zu diesem Zweck weist der Schnittblock 100 eine posterior angeordnete erste Befestigungsaufnahme 112 auf (siehe Fig. 7, 8). Der Referenzfuß 200 weist ein zu der ersten Befestigungsaufnahme 112 komplementäres Befestigungselement 202 auf (siehe Fig. 9, 10).

Die erste Befestigungsaufnahme 112 ist im Bereich der Blockunterseite 104 angeordnet. Bei der gezeigten Ausführungsform ist die erste Befestigungsaufnahme 112 ein von der Blockrückseite 101 auf die Blockvorderseite 102 reichender sowie mediolateral gerade längserstreckter Aufnahmeschlitz 113. Der Aufnahmeschlitz 113 weist bei der gezeigten Ausführungsform zwei getrennte Schlitzabschnitte 1131, 1132 auf, die auch als medialer Schlitzabschnitt 1132 und lateraler Schlitzabschnitt 1131 bezeichnet werden können. Die beiden Schlitzabschnitte 1131, 1132 sind in mediolateraler Richtung durch einen Steg 114 voneinander getrennt. Der laterale Schlitzabschnitt 1131 ist an seinem dem Steg 114 gegenüberliegenden Ende in lateraler Richtung offen. Der mediale Schlitzabschnitt 1132 ist an seinem dem Steg 114 gegenüberliegenden Ende in medialer Richtung offen.

Bei der gezeigten Ausführungsform kommt dem Aufnahmeschlitz 113 eine besonders vorteilhafte Mehrfachfunktion zu. Zum einen dient der Aufnahmeschlitz der form- und/oder kraftschlüssigen Aufnahme des komplementären Befestigungselements 202. Zum anderen dient der Aufnahmeschlitz 113 - in einem nicht an dem Schnittblock 100 befestigten Zustand des Referenzfußes 200 - als der posteriore Führungsschlitz 108. Mit anderen Worten ausgedrückt dient der posteriore Führungsschlitz 108 gleichzeitig als der Aufnahmeschlitz 113.

Das komplementäre Befestigungselement 202 (siehe Fig. 9, 10) ist komplementär zu dem Aufnahmeschlitz 113 gestaltet und vorliegend ein ebenes plattenförmiges Steckelement 203, welches zum form- und/oder kraftschlüssigen Einstecken in den Aufnahmeschlitz 113 eingerichtet ist. Dabei ist das Steckelement 203 vorliegend eben in Bezug auf die mediolaterale Achse und in Bezug auf die proximodistale Achse. In Entsprechung zu dem im weitesten Sinne zweigeteilten Aufnahmeschlitz 113 weist das Steckelement 203 zwei getrennte Steckabschnitte 2031, 2032 auf. Diese können auch als medialer Steckabschnitt 2032 und als lateraler Steckabschnitt 2031 bezeichnet werden. Die beiden Steckabschnitte 2031, 2032 sind in mediolateraler Richtung durch einen Spalt 204 voneinander getrennt. Dabei ist eine mediolaterale Erstreckung des Spalts 204 größer als die entsprechende Erstreckung des Stegs 114.

Zur Befestigung des Referenzfußes 200 an dem Schnittblock 100 wird das Steckelement 203 proximal in den Aufnahmeschlitz 113 eingesteckt. Hierbei werden der laterale Steckabschnitt 2031 in den lateralen Schlitzabschnitt 1131 und der mediale Steckabschnitt 2032 in den medialen Schlitzabschnitt 1132 eingeführt.

Bei der gezeigten Ausführungsform weist das Steckelement 203 wenigstens einen anteroposterior erstreckten Trennspalt 2033 auf, der unter Ausbildung eines elastisch federbeweglichen Federzungenabschnitts 2034 im Wesentlichen U-förmig längserstreckt ist. Der Federzungenabschnitt 2034 liegt in eingestecktem Zustand des Steckelements 203 federkraftbelastet an einer nicht näher bezeichneten Innenwand des Aufnahmeschlitzes 113 an. Hierbei drückt der Federzungenabschnitt 2034 anterior oder posterior gegen die besagte Innenwand. Dies führt zu einer erhöhten Reibung und folglich zu einer verbesserten und besonders zuverlässigen Befestigung des Referenzfußes 200. Der Trennspalt 2033 und der Federzungenabschnitt 2034 sind bei der gezeigten Ausführungsform an dem lateralen Steckabschnitt 2031 angeordnet, so dass auch von einem lateralen Trennspalt 2033 und einem lateralen Federzungenabschnitt 2034 gesprochen werden kann. Der mediale Steckabschnitt 2032 weist vorliegend eine dementsprechende Gestaltung mit einem (medialen) Trennspalt 2035 und einem (medialen) Federzungenabschnitt 2036 auf.

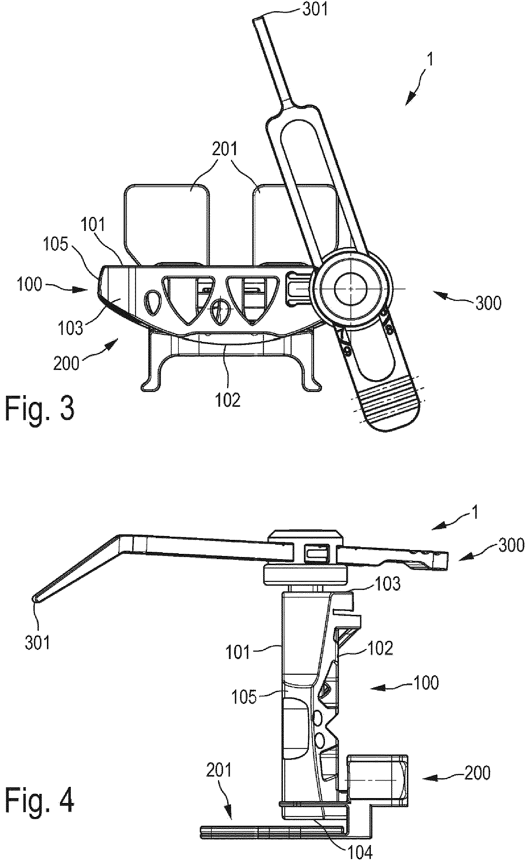

Bei der gezeigten Ausführungsform ist die Fußoberseite 201 an einem ebenen plattenförmigen Fortsatz 205 ausgebildet. Mit anderen Worten ausgedrückt bildet die nicht näher bezeichnete Oberseite des Fortsatzes 205 die Fußoberseite 201. Der Fortsatz 205 ist in Bezug auf die mediolaterale Achse und in Bezug auf die proximodistale Achse eben und ragt wenigstens abschnittsweise in proximaler Richtung über die Blockrückseite 101. Der Fortsatz 205 ist vorliegend planparallel zu dem Befestigungselement 203 orientiert. Der Fortsatz 205 ist anterior von dem Befestigungselement 203 beabstandet. In dem befestigten Zustand des Referenzfußes 200 untergreift der Fortsatz 205 die Blockunterseite 304 (siehe insbesondere Fig. 4).

Der Fortsatz 205 weist einen lateralen Fortsatzabschnitt 2051 und einen medialen Fortsatzabschnitt 2052 auf. Die beiden Fortsatzabschnitte 2051, 2052 sind in mediolateraler Richtung durch einen Spalt 206 voneinander getrennt. Der laterale Fortsatzabschnitt 2051 ist zur Kontaktierung der lateralen posterioren Kondyle KL vorgesehen. Dementsprechend ist der mediale Fortsatzabschnitt 2052 zur Kontaktierung der medialen posterioren Kondyle KM vorgesehen.

Zur Kompensation etwaiger Abnutzungen der posterioren Kondylen KL, KM weist das chirurgische Instrument 1 bei der gezeigten Ausführungsform zudem mehrere Kompensationselemente auf. Anhand der vorliegenden Zeichnungen ist lediglich ein Kompensationselement 400 der besagten mehreren Kompensationselemente gezeigt. Die unterschiedlichen Kompensationselemente unterscheiden sich hinsichtlich ihrer anteroposterioren Dicke und sind jeweils an der Fußoberseite 201 anbringbar. Vorliegend ist das Kompensationselement 400 an dem lateralen Fortsatzabschnitt 2051 angebracht. Zu diesem Zweck sind bei unterschiedlichen Ausgestaltungen unterschiedliche Fügeverbindungen zwischen dem Kompensationselement 400 (den mehreren unterschiedlichen Kompensationselementen) und der Fußoberseite 201 denkbar. Vorliegend ist das Kompensationselement 400 auf nicht näher gezeigte Weise auf den lateralen Fortsatzabschnitt 2051 aufgesteckt. Durch die Anbringung des Kompensationselements 400 an dem lateralen Fortsatzabschnitt 2051 kann eine Abnutzung der lateralen posterioren Kondyle KL dahingehend kompensiert werden, dass trotz der besagten Abnutzung eine anforderungsgerechte, dem Kinematic Alignment-Ansatz gemäße, rotatorische Ausrichtung des chirurgischen Instruments 1 um die proximodistale Achse gewährleistet ist.

Zur Verdeutlichung, dass das chirurgische Instrument 1 mehrere unterschiedliche Kompensationselemente aufweist, ist das Kompensationselement 400 in Fig. 9 mit weiteren in Klammern gesetzten Bezugszeichen 400', 400", 400‴ bezeichnet. Die besagten Bezugszeichen beziehen sich auf ein nicht gesondert zeichnerisch dargestelltes erstes Kompensationselement 400', zweites Kompensationselement 400" und drittes Kompensationselement 400‴. Die anteroposteriore Dicke des ersten Kompensationselements 400' ist kleiner als diejenige des zweiten Kompensationselements 400" und des dritten Kompensationselements 400‴. Die anteroposteriore Dicke des zweiten Kompensationselements 400" ist größer als diejenige des ersten Kompensationselements 400' und kleiner als diejenige des dritten Kompensationselements 400‴. Die anteroposteriore Dicke des dritten Kompensationselements 400‴ ist größer als diejenige des ersten Kompensationselements 400' und des zweiten Kompensationselements 400".

Bei der gezeigten Ausführungsform weist der Referenzfuß 200 zudem einen Griffabschnitt 207 auf. Der Griffabschnitt 207 ist distal an dem Referenzfuß 200 angeordnet und weist eine laterale Grifffläche 2071, eine mediale Grifffläche 2072 und eine Anschlagfläche 2073 auf. Der Griffabschnitt 207 dient einer vereinfachten manuellen Handhabung des Referenzfußes 200. Das Befestigungselement 202 ragt in proximaler Richtung von dem Griffabschnitt 207 ab. Der Fortsatz 205 ragt in proximaler Richtung von dem Griffabschnitt 207 ab. Die laterale Grifffläche 2071 ist im Wesentlichen lateral orientiert, so dass die entsprechende Flächennormale in etwa parallel zur mediolateralen Achse ausgerichtet ist und in laterale Richtung weist. Entsprechendes gilt mutatis mutandis für die mediale Grifffläche 2072. Zur Befestigung und zum Lösen des Referenzfußes wird der Griffabschnitt 207 zwischen den Fingern einer Hand gegriffen, wobei beispielsweise der Daumen an der lateralen Grifffläche 2071 und der Zeigefinger an der medialen Grifffläche 2072 anliegt. Die Anschlagfläche 2073 ist proximal orientiert und dient in erster Linie einer Begrenzung der Einstecktiefe des Steckelements 203 in den Aufnahmeschlitz 113. In eingestecktem Zustand kontaktiert die Anschlagfläche 2073 die Blockvorderseite 102.

Zur lösbaren Verbindung des Tasters 300 mit dem Schnittblock 100 weist letzterer eine anterior angeordnete zweite Befestigungsaufnahme 115 auf. Der Taster 300 weist ein zu der zweiten Befestigungsaufnahme 115 komplementäres Befestigungselement 302 auf. Die zweite Befestigungsaufnahme 115 und das Befestigungselement 302 des Tasters 300 sind form- und/oder kraftschlüssig miteinander verbindbar bzw. verbunden.

Wie insbesondere anhand der Fig. 7 und 8 gezeigt ist, weist die zweite Befestigungsaufnahme vorliegend 115 eine Rastaufnahme 116 und eine Steckaufnahme 117 auf.

Die Rastaufnahme 116 ist in posteriorer Richtung in die Blockoberseite 103 eingesenkt und ausgehend von der medialen Blockaußenseite 106 mediolateral längs in den Schnittblock 100 hineinerstreckt. Dabei ist die Rastaufnahme 116 in anteriorer Richtung (nach oben) offen. Die Rastaufnahme 116 ist folglich im weitesten Sinne taschenförmig ausgebildet.

Die Steckaufnahme 117 ist ausgehend von der medialen Blockaußenseite 106 in lateraler Richtung in den Schnittblock 100 eingebracht. Vorliegend weist die Steckaufnahme 117 einen kreiszylindrischen Querschnitt auf und kann als Bohrung bezeichnet werden. Bei einer zeichnerisch nicht dargestellten Ausführungsform sind sowohl die Rastaufnahme 116 als auch die Steckaufnahme 117 lateral an dem Schnittblock 100 angebracht. Zudem ist es denkbar, dass der Schnittblock sowohl medial als auch lateral eine zweite Befestigungsaufnahme aufweist. Die Steckaufnahme 117 ist posterior von der Rastaufnahme 116 beabstandet.

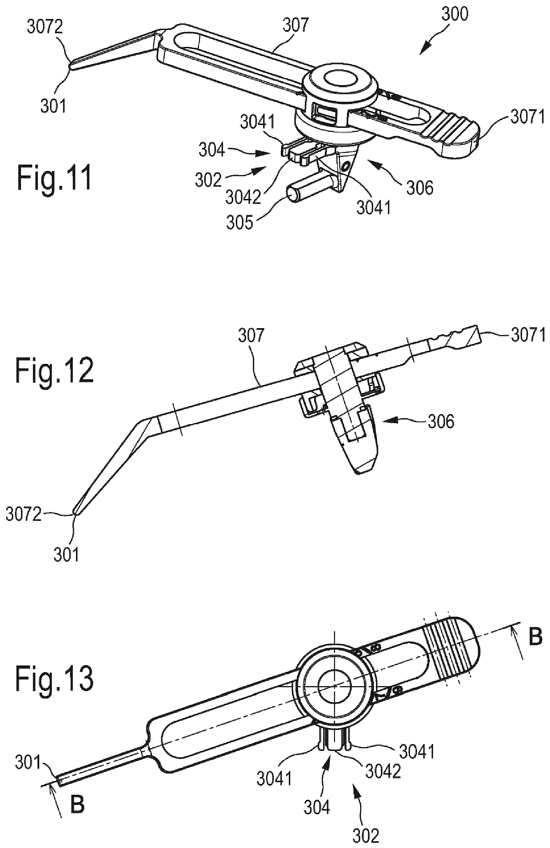

Das komplementäre Befestigungselement 302 des Tasters 300 weist einen Rastabschnitt 304 und einen Steckabschnitt 305 auf (siehe Fig. 11).

Der Rastabschnitt 304 ist komplementär zu der Rastaufnahme 116 gestaltet und weist vorliegend zwei elastische Federschenkel 3041 auf. Die elastischen Federschenkel 3041 liegen einander in Bezug auf die proximodistale Achse gegenüber und sind entlang derselben elastisch federbeweglich. In Bezug auf die proximodistale Achse zwischen den beiden Federschenkeln 3041 weist der Rastabschnitt 304 einen Führungszapfen 3042 auf.

Der Steckabschnitt 305 ist komplementär zu der Steckaufnahme 117 gestaltet und vorliegend folglich stift-, zapfen- und/oder bolzenförmig. Dabei ist der Steckabschnitt 305 mediolateral gerade längserstreckt.

In befestigtem Zustand des Tasters 300 ist der Steckabschnitt 305 koaxial zu der Steckaufnahme 117 ausgerichtet und axial in dieselbe eingesteckt. Dementsprechend ist der Rastabschnitt 304 mit der Rastaufnahme 116 verrastet. Hierbei sind die beiden Federschenkel 3041 formschlüssig mit einer nicht näher bezeichneten Innenkontur der Rastaufnahme 116 verrastet. Die vorliegende Gestaltung der zweiten Befestigungsaufnahme 115 und des komplementären Befestigungselements 302 bewirkt insbesondere eine verbesserte Momentenabstützung um die mediolaterale Achse. Hierdurch wird einer unerwünschten Relativbewegung der Tasterspitze 301 in Bezug auf den Schnittblock 100 entgegengewirkt.

Bei der gezeigten Ausführungsform ist die zweite Befestigungsaufnahme 115 Bestandteil eines Universalbefestigungssystems und zur Verbindung mit weiteren zeichnerisch nicht näher dargestellten Instrumentenkomponenten eingerichtet. Hierdurch kann anstelle des Tasters 300 wahlweise eine ebenfalls mit einem komplementären Befestigungselement ausgestattete weitere Instrumentenkomponente mit dem Schnittblock 100 lösbar verbunden werden. Als Instrumentenkomponente kommt insbesondere eine chirurgische Navigationsvorrichtung oder dergleichen infrage.

Bei der gezeigten Ausführungsform ist die Tasterspitze 301 relativ zu dem Schnittblock 100 verlagerbar. Zu diesem Zweck weist der Taster 300 vorliegend einen Lagerblock 306 und ein an demselben beweglich gelagertes Tasterelement 307 auf.

Das Tasterelement 307 ist zwischen einem ersten Ende 3071 und einem zweiten Ende 3072 längserstreckt, wobei die Tasterspitze 301 an dem zweiten Ende 3072 ausgebildet ist. Das Tasterelement 307 ist entlang seiner Längsrichtung relativ zu dem Lagerblock 306 linearbeweglich an demselben geführt. Zudem ist das Tasterelement 307 um eine Schwenkachse relativ zu dem Lagerblock 306 schwenkbeweglich. Die nicht näher bezeichnete Schwenkachse ist parallel zu der anteroposterioren Achse orientiert. Der Lagerblock 306 weist das komplementäre Befestigungselement 302 und somit sowohl den Rastabschnitt 304 als auch den Steckabschnitt 305 auf.

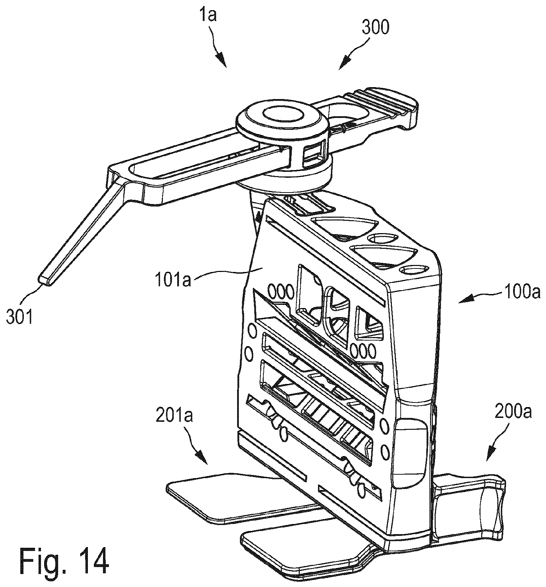

Fig. 14 zeigt eine weitere Ausführungsform eines erfindungsgemäßen chirurgischen Instruments 1a. Die Funktion und der Aufbau des chirurgischen Instruments 1a sind weitestgehend identisch mit der Funktion und dem Aufbau des chirurgischen Instruments 1 nach den Fig. 1 bis 6. Zur Vermeidung von Wiederholungen werden nachfolgend lediglich wesentliche Unterschiede des chirurgischen Instruments 1a gegenüber dem chirurgischen Instrument 1 erläutert. Funktionsgleiche Bauteile und/oder Abschnitte des chirurgischen Instruments 1a werden nicht gesondert erläutert. Stattdessen wird ausdrücklich auf die Offenbarung zu dem chirurgischen Instrument 1 verwiesen und Bezug genommen.

Das chirurgische Instrument 1a unterscheidet sich im Wesentlichen durch eine unterschiedliche Art und Weise der Befestigung des Referenzfußes 200a an dem Schnittblock 100a. Dementgegen sind der Taster 300 und seine Befestigung an dem Schnittblock 100a identisch zu dem Taster 300 des chirurgischen Instruments 1.

Der Schnittblock 100a weist eine erste Befestigungsaufnahme 112a auf. Diese ist vorliegend eine in die Blockvorderseite 102a proximal eingesenkte Aufnahmetasche 113a. Die Aufnahmetasche 113a ist anterior von dem posterioren Führungsschlitz 108a des Schnittblocks 108a beabstandet und in etwa mittig zwischen den Blockaußenseiten 105a, 106a angeordnet.

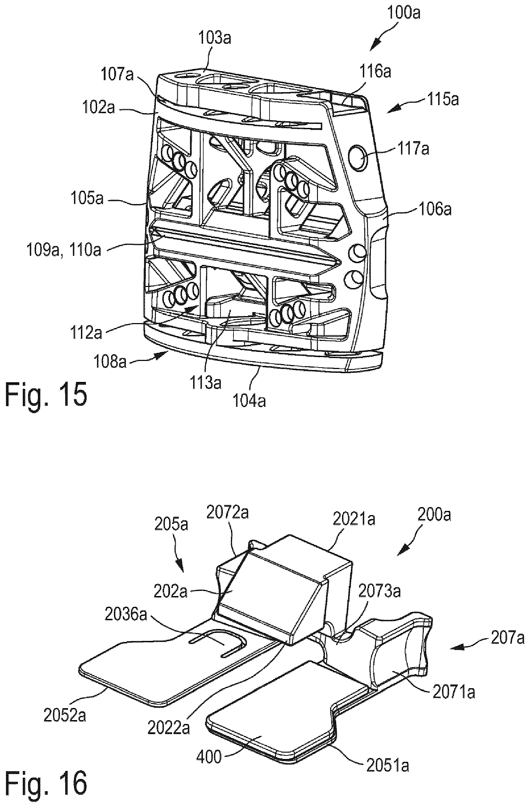

Der Referenzfuß 200a (Fig. 16) weist ein zu der Aufnahmetasche 113a komplementäres Befestigungselement 202a auf. Das Befestigungselement 202a ist zum Ausbilden einer Rast- und/oder Klemmverbindung mit der Aufnahmetasche 13a eingerichtet. Bei der gezeigten Ausführungsform ist das Befestigungselement 202a keilförmig gestaltet und zwischen einem distalen Ende 2021a und einem proximalen Ende 2022a längserstreckt. Dabei ist das Befestigungselement 202a in Richtung des proximalen Stirnendes 2022a keilförmig abgeflacht.

Zur lösbaren Verbindung mit dem Schnittblock 100a wird das Befestigungselement 202a mit dem proximalen Stirnende 2022a voran in die Aufnahmetasche 113a eingeführt. Hierbei kommt die besagte Rast- und/oder Klemmverbindung zustande.

Im Übrigen weist der Referenzfuß 200a eine mit dem Referenzfuß 200 des chirurgischen Instruments 1 nach den Fig. 1 bis 13 im Wesentlichen übereinstimmenden Aufbau auf. Insoweit sind wiederum ein lateraler Fortsatzabschnitt 2051a und ein medialer Fortsatzabschnitt 2052a vorhanden. Vorliegend ist der mediale Fortsatzabschnitt 2052a mit einem Federzungenabschnitt 2036a versehen, der zur reibschlüssigen Unterstützung der Befestigung des Referenzfußes federkraftbelastet gegen die Blockunterseite 104a drücken kann. Zur Kompensation etwaiger kondylärer Abnutzungen ist ein Kompensationselement 400 auf den lateralen Fortsatzabschnitt 2051a aufgesteckt, was selbstverständlich nicht zwingend ist.

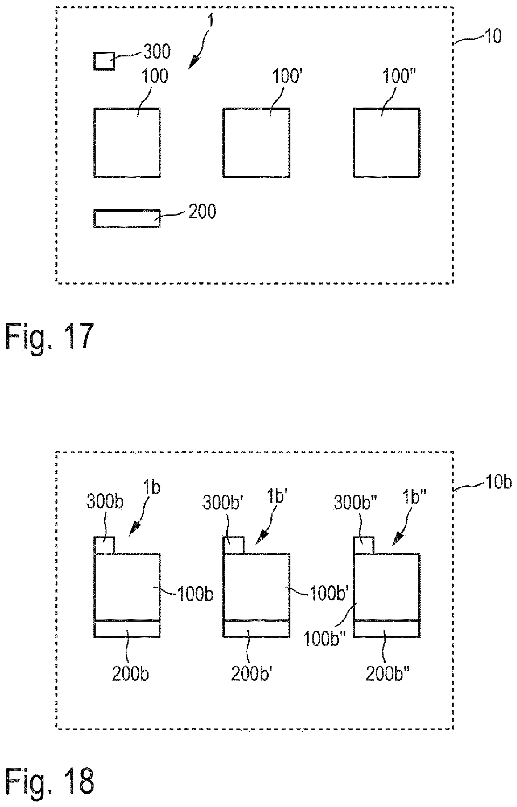

Fig. 17 zeigt eine Ausführungsform eines erfindungsgemäßen chirurgischen Instrumentensystems 100. Das chirurgische Instrumentensystem 100 weist das chirurgische Instrument 1 nach den Fig. 1 bis 6 auf und umfasst zudem weitere Schnittblöcke 100', 100". Die Schnittblöcke 100, 100', 100" werden nachfolgend noch als erster Schnittblock 100, zweiter Schnittblock 100' und dritter Schnittblock 100" bezeichnet. Die Schnittblöcke 100, 100', 100" sind hinsichtlich ihrer Abmessungen unterschiedlich und zur Verwendung an unterschiedlich großen Femurknochen vorgesehen. Abgesehen von den unterschiedlichen Abmessungen weisen der zweite Schnittblock 100' und der dritte Schnittblock 100" eine mit dem ersten Schnittblock 100 identische Funktion und Gestaltung auf.

Der Referenzfuß 200 und der Taster 300 sind jeweils an jedem der Schnittblöcke 100, 100', 100" wahlweise anbringbar. Dies mittels der vorbeschriebenen Befestigungsaufnahmen und Befestigungselemente.

Fig. 18 zeigt ein nicht erfindungsgemäßes chirurgisches Instrumentensystem 10b. Das chirurgische Instrumentensystem 10b weist ein chirurgisches Instrument 1b auf, welches wiederum einen Schnittblock 10b, einen Referenzfuß 200b und einen Taster 300b umfasst. Das chirurgische Instrument 1b ist hinsichtlich seiner Funktion und Gestaltung im Wesentlichen identisch mit dem chirurgischen Instrument 1 nach den Fig. 1 bis 6. Unterschiedlich sind in erster Linie die Art und Weise der Befestigung des Referenzfußes 200b und des Tasters 300b jeweils an dem Schnittblock 100b. Anstelle einer lösbaren Befestigung ist bei dem nicht erfindungsgemäßen chirurgischen Instrument 1b eine jeweils feste Anbringung des Referenzfußes 200b und des Tasters 300b an dem Schnittblock 100b vorgesehen. Fest meint, dass der Referenzfuß 200b und der Taster 300b jeweils nicht zum wahlweisen Anbringen und Entfernen an dem Schnittblock 100b eingerichtet sind. Stattdessen sind nicht näher gezeigte und bezeichnete Fügeverbindungen zwischen dem Referenzfuß 200b und dem Schnittblock 100b einerseits und dem Taster 300b und dem Schnittblock 100b andererseits vorhanden.

Das chirurgische Instrumentensystem 10b weist zudem weitere chirurgische Instrumente 1b', 1b" auf. Die chirurgischen Instrumente 1b, 1b', 1b" werden nachfolgend auch als erstes chirurgisches Instrument 1b, zweites chirurgisches Instrument 1b' und drittes chirurgisches Instrument 1b" bezeichnet. Die chirurgischen Instrumente 1b, 1b', 1b" sind hinsichtlich ihrer Abmessungen unterschiedlich und zur Verwendung an unterschiedlich großen Femurknochen vorgesehen. Dabei unterscheiden sich die chirurgischen Instrumente 1b, 1b', 1b" wenigstens im Hinblick auf eine mediolaterale Abmessung des jeweiligen Schnittblocks 100b, 100b', 100b". Zudem kann der jeweilige Taster 300b, 300b', 300b" unterschiedliche Abmessungen, insbesondere entlang der proximodistalen Achse, aufweisen. Entsprechendes kann für den jeweiligen Referenzfuß 200b, 200b', 200b" gelten.

## Patentansprüche

1. Chirurgisches Instrument (1, 1a) zur Verwendung bei einer Kniegelenkersatzoperation, aufweisend
einen Schnittblock (100, 100a) mit einer proximal orientierten Blockrückseite (101, 101a), welche zur Anlage an einer distalen Stirnfläche (S) eines resezierten Femurs (F) eingerichtet ist, und mit wenigstens einem von der Blockrückseite (101, 101a) auf eine distal gegenüberliegende Blockvorderseite (102, 102a) reichenden sowie mediolateral längserstreckten Führungsschlitz (107, 108, 109, 110; 107a, 108a, 109a, 110a), welcher zur Aufnahme und Führung eines Sägeblatts eingerichtet ist,
einen Referenzfuß (200, 200a), welcher im Bereich einer posterioren Blockunterseite (104, 104a) des Schnittblocks (100, 100a) an demselben anbringbar oder angebracht ist und eine anterior orientierte Fußoberseite (201, 201a) aufweist, welche - in einem angebrachten Zustand des Referenzfußes (200, 200a) - proximal über die Blockrückseite (101, 101a) ragt und zur Anlage an posterioren Kondylen (KL, KM) des Femurs (F) eingerichtet ist, und aufweisend
einen Taster (300), welcher im Bereich einer der Blockunterseite (104, 104a) anterior gegenüberliegenden Blockoberseite (103, 103a) des Schnittblocks (100, 100a) an demselben anbringbar oder angebracht ist und eine Tasterspitze (301) aufweist, welche - in einem angebrachten Zustand des Tasters (300) - proximal über die Blockrückseite (101, 101a) ragt und zur Anlage an einer anterioren Fläche (A) des Femurs (F) eingerichtet ist,
wobei der Schnittblock (100, 100a) eine anterior angeordnete zweite Befestigungsaufnahme (115, 115a) aufweist, wobei der Taster (300) ein komplementäres Befestigungselement (302) aufweist, und wobei die zweite Befestigungsaufnahme (115, 115a) und das Befestigungselement (302) des Tasters (300) lösbar form- und/oder kraftschlüssig miteinander verbindbar oder verbunden sind,
wobei die zweite Befestigungsaufnahme (115, 115a) eine Rastaufnahme (116, 116a) aufweist, welche in die Blockoberseite (103, 103a) posterior eingesenkt ist, und das Befestigungselement (302) des Tasters (300) einen komplementären Rastabschnitt (304) aufweist, welcher unter Ausbildung einer lösbaren Schnapp-Verbindung mit der Rastaufnahme des Schnittblocks (100, 100a) zusammenwirkt, **dadurch gekennzeichnet, dass** die Rastaufnahme mediolateral längserstreckt sowie medial oder lateral einends offen ist.

2. Chirurgisches Instrument (1, 1a) nach Anspruch 1, **dadurch gekennzeichnet, dass** der Schnittblock (100, 100a) eine posterior angeordnete erste Befestigungsaufnahme (112, 112a) aufweist, dass der Referenzfuß (200, 200a) ein komplementäres Befestigungselement (202, 202a) aufweist, und dass die erste Befestigungsaufnahme (112, 112a) und das Befestigungselement (202, 202a) lösbar form- und/oder kraftschlüssig miteinander verbindbar oder verbunden sind.

3. Chirurgisches Instrument (1) nach Anspruch 2, **dadurch gekennzeichnet, dass** die erste Befestigungsaufnahme (112) ein von der Blockrückseite (101) auf die Blockvorderseite (102) reichender sowie mediolateral gerade längserstreckter Aufnahmeschlitz (113) ist, und dass das Befestigungselement (202) ein ebenes plattenförmiges Steckelement (203) ist, welches zum form- und/oder kraftschlüssigen Einstecken in den Aufnahmeschlitz (113) eingerichtet ist.

4. Chirurgisches Instrument (1) nach Anspruch 3, **dadurch gekennzeichnet, dass** das Steckelement (203) wenigstens einen anteroposterior erstreckten Trennspalt (2033, 2035) aufweist, der unter Ausbildung eines elastisch federbeweglichen Federzungenabschnitts (2034, 2036) im Wesentlichen U-förmig längserstreckt ist, wobei der Federzungenabschnitt (2034, 2036) - in dem angebrachten Zustand des Referenzfußes (200) - federkraftbelastet gegen eine Innenwand des Aufnahmeschlitzes (113) drückt.

5. Chirurgisches Instrument (1) nach Anspruch 3 oder 4, **dadurch gekennzeichnet, dass** der Aufnahmeschlitz (113) zwei getrennte Schlitzabschnitte (1131, 1132) aufweist, welche in mediolateraler Richtung durch einen Steg (114) voneinander getrennt sind, und dass das Steckelement (203) zwei getrennte Steckabschnitte (2031, 2032) aufweist, welche in mediolateraler Richtung durch einen Spalt (204) voneinander getrennt sind.

6. Chirurgisches Instrument (1) nach einem der Ansprüche 3 bis 5, **dadurch gekennzeichnet, dass** der Aufnahmeschlitz (113) - in einem nicht angebrachten Zustand des Referenzfußes (200) - zur Aufnahme und Führung des Sägeblatts eingerichtet ist und einen posterioren Führungsschlitz (108) des Schnittblocks (100) bildet.

7. Chirurgisches Instrument (1a) nach Anspruch 2, **dadurch gekennzeichnet, dass** die erste Befestigungsaufnahme (112a) eine in die Blockvorderseite proximal eingesenkte Aufnahmetasche (113a) ist, und dass das Befestigungselement (202a) zum Ausbilden einer Rast- und/oder Klemmverbindung mit der Aufnahmetasche (113a) eingerichtet ist.

8. Chirurgisches Instrument (1, 1a) nach einem der vorhergehenden Ansprüche, **dadurch gekennzeichnet, dass** der Referenzfuß (200, 200a) einen, insbesondere posterior von dem Befestigungselement (202, 202a), beabstandeten, ebenen plattenförmigen Fortsatz (205, 205a) aufweist, dessen Oberseite die Fußoberseite (201, 201a) bildet.

9. Chirurgisches Instrument (1, 1a) nach einem der Ansprüche 2 bis 8, **dadurch gekennzeichnet, dass** der Referenzfuß (200, 200a) einen distal angeordneten Griffabschnitt (207, 207a) aufweist, von welchem das Befestigungselement (202, 202a) und/oder der Fortsatz (205, 205a) in proximaler Richtung abragt, wobei der Griffabschnitt (207, 207a) eine lateral orientierte erste Grifffläche (2071, 2071a), eine medial orientierte zweite Grifffläche (2072, 2072a) und eine proximal orientierte Anschlagfläche (2073, 2073a) aufweist.

10. Chirurgisches Instrument (1, 1a) nach einem der vorhergehenden Ansprüche, **dadurch gekennzeichnet, dass** die zweite Befestigungsaufnahme (115, 115a) als Bestandteil eines Universalbefestigungssystems gestaltet und zur Verbindung mit weiteren Instrumentenkomponenten, bevorzugt einer chirurgischen Navigationsvorrichtung, eingerichtet ist.

11. Chirurgisches Instrument (1, 1a) nach einem der vorhergehenden Ansprüche, **dadurch gekennzeichnet, dass** die zweite Befestigungsaufnahme (115, 115a) eine Steckaufnahme (117, 117a) aufweist, welche mediolateral in eine Blockaußenseite (105, 106; 105a, 106a) des Schnittblocks (100, 100a) längserstreckt ist, und dass das Befestigungselement (302) des Tasters (300) einen komplementären Steckabschnitt (305) aufweist, welcher unter Ausbildung einer lösbaren Steckverbindung mit der Steckaufnahme (117, 117a) zusammenwirkt.

12. Chirurgisches Instrument (1, 1a) nach einem der vorhergehenden Ansprüche, **dadurch gekennzeichnet, dass** mehrere unterschiedliche Kompensationselemente (400, 400', 400", 400‴) mit unterschiedlicher anteroposteriorer Dicke vorhanden sind, wobei die unterschiedlichen Kompensationselemente (400, 400', 400", 400‴) jeweils an der Fußoberseite (201, 201a) anbringbar, vorzugsweise auf dieselbe aufsteckbar, und zur Kompensation unterschiedlich stark ausgeprägter Abnutzungen der posterioren Kondylen (KL, KM) eingerichtet sind.

13. Chirurgisches Instrumentensystem (10) zur Verwendung bei einer Kniegelenkersatzoperation, mit wenigstens einem chirurgischen Instrument (1, 1a) nach einem der vorhergehenden Ansprüche, wobei der Referenzfuß (200, 200a) und der Taster (300) jeweils lösbar an dem Schnittblock (100, 101a) angebracht sind, und mit weiteren unterschiedlichen Schnittblöcken (100', 100"), welche sich im Hinblick auf wenigstens eine, insbesondere anteroposteriore und/oder mediolaterale, Abmessung unterscheiden, wobei der Referenzfuß (200, 200a) und der Taster (300) jeweils an jedem der weiteren unterschiedlichen Schnittblöcke (100', 100") anbringbar sind.

## Claims

1. Surgical instrument (1, 1a) for use in a knee joint replacement operation, having
a cutting block (100, 100a) with a proximally oriented block rear face (101, 101a) which is configured to bear on a distal end face (S) of a resected femur (F), and with at least one guide slot (107, 108, 109, 110; 107a, 108a, 109a, 110a) which reaches from the block rear face (101, 101a) to a distally opposite block front face (102, 102a) and is elongate in a mediolateral direction and is configured to receive and guide a saw blade,
a reference foot (200, 200a) which can be mounted or is mounted on the cutting block (100, 100a), in the region of a posterior block lower face (104, 104a) of the cutting block (100, 100a), and has an anteriorly oriented foot upper face (201, 201a) which, in a mounted state of the reference foot (200, 200a), protrudes proximally beyond the block rear face (101, 101a) and is configured to bear on posterior condyles (KL, KM) of the femur (F), and having
a probe (300) which can be mounted or is mounted on the cutting block (100, 100a), in the region of a block upper face (103, 103a) lying anteriorly opposite the block lower face (104, 104a), and has a probe tip (301) which, in a mounted state of the probe (300), protrudes proximally beyond the block rear face (101, 101a) and is configured to bear on an anterior face (A) of the femur (F),
wherein the cutting block (100, 100a) has an anteriorly arranged second fastening recess (115, 115a), wherein the probe (300) has a complementary fastening element (302), and wherein the second fastening recess (115, 115a) and the fastening element (302) of the probe (300) are releasably connectable or connected to each other with form-fit and/or force-fit engagement,
wherein the second fastening recess (115, 115a) has a latch recess (116, 116a) which is sunk posteriorly into the block upper face (103, 103a), and wherein the fastening element (302) of the probe (300) has a complementary latch portion (304) which interacts with the latch recess of the cutting block (100, 100a) to form a releasable snap-fit connection,
**characterized in that** the latch recess is elongate in the mediolateral direction and is open at a medial or lateral end.

2. Surgical instrument (1, 1a) according to Claim 1, **characterized in that** the cutting block (100, 100a) has a posteriorly arranged first fastening recess (112, 112a), **in that** the reference foot (200, 200a) has a complementary fastening element (202, 202a), and **in that** the first fastening recess (112, 112a) and the fastening element (202, 202a) are releasably connectable or connected to each other with form-fit and/or force-fit engagement.

3. Surgical instrument (1) according to Claim 2, **characterized in that** the first fastening recess (112) is a receiving slot (113) which reaches from the block rear face (101) to the block front face (102) and which is straight and elongate in the mediolateral direction, and **in that** the fastening element (202) is a flat plate-shaped plug element (203) which is configured to be plugged with form-fit and/or force-fit engagement into the receiving slot (113).

4. Surgical instrument (1) according to Claim 3, **characterized in that** the plug element (203) has at least one separating gap (2033, 2035) which extends in a anteroposterior direction and which is substantially U-shaped in longitudinal extent so as to form an elastically resiliently movable spring tongue portion (2034, 2036), wherein the spring tongue portion (2034, 2036), in the mounted state of the reference foot (200), is biased against an inner wall of the receiving slot (113).

5. Surgical instrument (1) according to Claim 3 or 4, **characterized in that** the receiving slot (113) has two separate slot portions (1131, 1132) which are separated from each other in the mediolateral direction by a web (114), and **in that** the plug element (203) has two separate plug portions (2031, 2032) which are separated from each other in the mediolateral direction by a gap (204).

6. Surgical instrument (1) according to one of Claims 3 to 5, **characterized in that** the receiving slot (113), in a non-mounted state of the reference foot (200), is configured to receive and guide the saw blade and forms a posterior guide slot (108) of the cutting block (100).

7. Surgical instrument (1a) according to Claim 2, **characterized in that** the first fastening recess (112a) is a receiving pocket (113a) sunk proximally into the block front face, and **in that** the fastening element (202a) is configured to form a latching and/or clamping connection with the receiving pocket (113a).

8. Surgical instrument (1, 1a) according to one of the preceding claims, **characterized in that** the reference foot (200, 200a) forms a flat plate-shaped extension (205, 205a) which is spaced apart, in particular posteriorly, from the fastening element (202, 202a) and whose upper face forms the foot upper face (201, 201a).

9. Surgical instrument (1, 1a) according to one of Claims 2 to 8, **characterized in that** the reference foot (200, 200a) has a distally arranged grip portion (207, 207a) from which the fastening element (202, 202a) and/or the extension (205, 205a) protrudes in the proximal direction, wherein the grip portion (207, 207a) has a laterally oriented first grip surface (2071, 2071a), a medially oriented second grip surface (2072, 2072a) and a proximally oriented stop surface (2073, 2073a).

10. Surgical instrument (1, 1a) according to one of the preceding claims, **characterized in that** the second fastening recess (115, 115a) is designed as a constituent part of a universal fastening system and is configured for connection to further instrument components, preferably a surgical navigation device.

11. Surgical instrument (1, 1a) according to one of the preceding claims, **characterized in that** the second fastening recess (115, 115a) has a plug recess (117, 117a) which extends mediolaterally into a block outer face (105, 106; 105a, 106a) of the cutting block (100, 100a), and **in that** the fastening element (302) of the probe (300) has a complementary plug portion (305) which interacts with the plug recess (117, 117a) to form a releasable plug connection.

12. Surgical instrument (1, 1a) according to one of the preceding claims, **characterized in that** a plurality of different compensation elements (400, 400', 400", 400‴) with different anteroposterior thickness are present, wherein the different compensation elements (400, 400', 400", 400‴) are each configured to be mountable, preferably pluggable, onto the foot upper face (201, 201a) and to compensate for different degrees of wear of the posterior condyles (KL, KM).

13. Surgical instrument system (10) for use in a knee joint replacement operation, having at least one surgical instrument (1, 1a) according to one of the preceding claims, wherein the reference foot (200, 200a) and the probe (300) are each mounted releasably on the cutting block (100, 101a), and having further different cutting blocks (100', 100") which differ from one another in terms of at least one dimension, in particular an anteroposterior and/or mediolateral dimension, wherein the reference foot (200, 200a) and the probe (300) are each mountable on each of the further different cutting blocks (100', 100").

## Revendications

1. Instrument chirurgical (1, 1a) destiné à être utilisé lors d'une opération de remplacement de l'articulation du genou, présentant
un bloc de coupe (100, 100a) pourvu d'une face arrière (101, 101a) de bloc, orientée de manière proximale, qui est conçue pour s'appuyer contre une surface frontale (S) distale d'un fémur (F) réséqué, et d'au moins fente de guidage (107, 108, 109, 110 ; 107a, 108a, 109a, 110a) s'étendant longitudinalement de manière médio-latérale et allant de la face arrière (101, 101a) de bloc à une face avant (102, 102a) de bloc opposée de manière distale, laquelle fente est conçue pour recevoir et guider une lame de scie,
un pied de référence (200, 200a), qui peut être ou est monté dans la zone d'une face inférieure (104, 104a) postérieure du bloc de coupe (100, 100a) sur celui-ci et qui présente une face supérieure (201, 201a) de pied orientée de manière antérieure qui s'étend - dans un état monté du pied de référence (200, 200a) - de manière proximale au-delà de la face arrière (101, 101a) de bloc et qui est conçue pour s'appuyer contre le condyle postérieur (KL, KM) du fémur (F) et présentant un palpeur (300), qui peut être ou est monté dans la zone d'une face supérieure (103, 103a) du bloc de coupe (100, 100a) opposée de manière antérieure à la face inférieure (104, 104a) de bloc et qui présente une pointe (301) de palpeur qui s'étend - dans un état monté du palpeur (300) - de manière proximale au-delà de la face arrière (101, 101a) de bloc et qui est conçue pour s'appuyer contre une surface antérieure (A) du fémur (F),
le bloc de coupe (100, 100a) présentant un deuxième logement de fixation (115, 115a) agencé de manière antérieure, le palpeur (300) présentant un élément de fixation complémentaire (302) et le deuxième logement de fixation (115, 115a) et l'élément de fixation (302) du palpeur (300) pouvant être ou étant reliés l'un à l'autre de manière amovible par complémentarité de forme et/ou à force,
le deuxième logement de fixation (115, 115a) présentant un logement d'encliquetage (116, 116a) qui est enfoncé de manière postérieure dans la face supérieure (103, 103a) de bloc et l'élément de fixation (302) du palpeur (300) présentant une section d'encliquetage (304) complémentaire, qui coopère avec le logement d'encliquetage du bloc de coupe (100, 100a) avec formation d'une liaison à encliquetage amovible, **caractérisé en ce que** le logement d'encliquetage s'étend longitudinalement de manière médio-latérale et est ouvert sur une extrémité de manière médiale ou latérale.

2. Instrument chirurgical (1, 1a) selon la revendication 1, **caractérisé en ce que** le bloc de coupe (100, 100a) présente un premier logement de fixation (112, 112a) agencé de manière postérieure, **en ce que** le pied de référence (200, 200a) présente un élément de fixation (202, 202a) complémentaire et **en ce que** le premier logement de fixation (112, 112a) et l'élément de fixation (202, 202a) peuvent être ou sont reliés l'un à l'autre de manière amovible par complémentarité de forme et/ou à force.

3. Instrument chirurgical (1) selon la revendication 2, **caractérisé en ce que** le premier logement de fixation (112) est une fente de logement (113) allant de la face arrière (101) de bloc à la face avant (102) de bloc et s'étendant longitudinalement de manière médio-latérale et droite et **en ce que** l'élément de fixation (202) est un élément enfichable (203) en forme de place qui est conçu pour s'enficher par complémentarité de forme et/ou à force dans la fente de logement (113).

4. Instrument chirurgical (1) selon la revendication 3, **caractérisé en ce que** l'élément enfichable (203) présente au moins une fente de séparation (2033, 2035) s'étendant de manière antéro-postérieure, qui s'étend longitudinalement essentiellement en forme de U en formant une section (2034, 2036) à languette à ressort élastiquement mobile par ressort, la section (2034, 2036) à languette à ressort - dans l'état monté du pied de référence (200) - poussant sous l'effet d'une force de ressort contre une paroi interne de la fente de logement (113).

5. Instrument chirurgical (1) selon la revendication 3 ou 4, **caractérisé en ce que** la fente de logement (113) présente deux sections à fente (1131, 1132) séparées qui sont séparées l'une de l'autre dans la direction médio-latérale par une âme (114) et **en ce que** l'élément enfichable (203) présente deux sections enfichables (2031, 2032) séparées qui sont séparées l'une de l'autre dans la direction médio-latérale par un interstice (204).

6. Instrument chirurgical (1) selon l'une des revendications 3 à 5, **caractérisé en ce que** la fente de logement (113) - dans un état non monté du pied de référence (200) - est conçue pour recevoir et guider la lame de scie et forme une fente de guidage (108) postérieure du bloc de coupe (100).

7. Instrument chirurgical (1a) selon la revendication 2, **caractérisé en ce que** le premier logement de fixation (112a) est une poche de logement (113a) enfoncée de manière proximale dans la face avant de bloc et **en ce que** l'élément de fixation (202a) est conçu pour former une liaison à encliquetage et/ou à serrage avec la poche de logement (113a).

8. Instrument chirurgical (1, 1a) selon l'une des revendications précédentes, **caractérisé en ce que** le pied de référence (200, 200a) présente un prolongement (205, 205a) plan en forme de plaque, en particulier écarté de manière postérieure de l'élément de fixation (202, 202a), dont la face supérieure forme la face supérieure (201, 201a) du pied.

9. Instrument chirurgical (1, 1a) selon l'une des revendications 2 à 8, **caractérisé en ce que** le pied de référence (200, 200a) présente une section de préhension (207, 207a) agencée de manière distale, à partir de laquelle s'étend l'élément de fixation (202, 202a) et/ou le prolongement (205, 205a) dans la direction proximale, la section de préhension (207, 207a) présentant une première surface de préhension (2071, 2071a) orientée latéralement, une deuxième surface de préhension (2072, 2072a) orientée de manière médiale et une surface de butée (2073, 2073a) orientée de manière proximale.

10. Instrument chirurgical (1, 1a) selon l'une des revendications précédentes, **caractérisé en ce que** le deuxième logement de fixation (115, 115a) est réalisé comme composant d'un système de fixation universel et conçu pour une liaison avec d'autres composants d'instrument, de préférence d'un dispositif de navigation chirurgical.

11. Instrument chirurgical (1, 1a) selon l'une des revendications précédentes, **caractérisé en ce que** le deuxième logement de fixation (115, 115a) présente un logement d'enfichage (117, 117a) qui s'étend longitudinalement de manière médio-latérale dans une face externe (105, 106 ; 105a, 106a) du bloc de coupe (100, 100a) et **en ce que** l'élément de fixation (302) du palpeur (300) présente une section enfichable (305) complémentaire qui coopère avec le logement d'enfichage (117, 117a) en formant une liaison enfichée amovible.

12. Instrument chirurgical (1, 1a) selon l'une des revendications précédentes, **caractérisé en ce qu'**un ou plusieurs éléments de compensation (400, 400', 400", 400‴) différents d'une épaisseur antéro-postérieure différente sont présents, les différents éléments de compensation (400, 400', 400", 400"') pouvant à chaque être montés sur la face supérieure (201, 201a) de pied, de préférence enfichés sur celle-ci et étant conçus pour la compensation d'usures marquées à des degrés divers du condyle postérieur (KL, KM).

13. Instrument chirurgical (10) destiné à être utilisé lors d'une opération de remplacement de l'articulation du genou, présentant au moins un instrument chirurgical (1, 1a) selon l'une des revendications précédentes, le pied de référence (200, 200a) et le palpeur (300) étant à chaque fois montés de manière amovible sur le bloc de coupe (100, 101a) et présentant différents autres blocs de coupe (100', 100"), qui se distinguent eu égard à au moins une dimension, en particulier antéro-postérieure et/ou médio-latérale, le pied de référence (200, 200a) et le palpeur (300) pouvant à chaque fois être montés sur chacun des différents autres blocs de coupe (100', 100").
